## Europäisches Patentamt
## European Patent Office
## Office européen des brevets

(19)

(11) Numéro de publication: **0 023 860**
**B1**

(12) **FASCICULE DE BREVET EUROPEEN**

(45) Date de publication du fascicule du brevet: **22.12.82**

(51) Int. Cl.³: **C 12 N 9/64, A 61 K 37/54**

(21) Numéro de dépôt: **80401111.2**

(22) Date de dépôt: **25.07.80**

(54) **Procédé perfectionné de préparation d'activateur du plasminogène, activateur ainsi obtenu et médicament le contenant.**

(30) Priorité: **27.07.79 FR 7919432**

(43) Date de publication de la demande:
**11.02.81 Bulletin 81/6**

(45) Mention de la délivrance du brevet:
**22.12.82 Bulletin 82/51**

(84) Etats contractants désignés:
**AT BE CH DE FR GB IT LI LU NL SE**

(56) Documents cités:
**FR - A - 2 252 842**
**FR - A - 2 310 133**
**FR - A - 2 347 048**

**CHEMICAL ABSTRACTS, vol. 89, no. 7 14 août 1978, page 360, réf. no. 57189k Columbus, Ohio, US**
**P. WALLEN: "Activation of plasminogen with urokinase and tissue activator"**

**CHEMICAL ABSTRACTS, vol. 77, no. 17, 23 october 1972, page 259, réf. no. 112034k Columbus, Ohio, US**

(73) Titulaire: **PIERRE FABRE S.A.**
**125, rue de la Faisanderie**
**F-75116 Paris (FR)**

(72) Inventeur: **Dussourd d'Hinterland, Lucien, Dr.**
**Domaine de Plombières Avenue de Lautrec**
**F-81100 Castres (FR)**
Inventeur: **Normier, Gérard**
**23, Rue Francis Poullenc**
**F-81100 Castres (FR)**

(74) Mandataire: **Corre, Jacques Denis Paul et al,**
**Cabinet Regimbeau 26, Avenue Kléber**
**F-75116 Paris (FR)**

Courier Press, Leamington Spa, England.

(56) Documents cités:
S. THORSEN et al.: "Differences in the binding to fibrin of urokinase and tissue plasminogen activator".

ACTA CHEMICA SCANDINAVIA, vol. 12, no. 9, 1958
Copenhagen (DK)
M. LASSEN: "Adsorption of plasminogen activator to fibrin", pages 1825—29.

CHEMICAL ABSTRACTS, vol. 84, no. 13, 29 mars 1976, page 229, rèf. no. 86283p
Columbus, Ohio US
F. R. MATTHIAS et al.: "Isolation of fibrinogen from plasma by affinity chromatography on insolubilized fibrinogen (FG-ag.) and insolubilized fibrinmonomer (FM-ag)".

CHEMICAL ABSTRACTS, vol. 92, no. 21, 26 mai 1980, page 251, ref. no. 176523m
Columbus, Ohio, US
S. A. CEDERHOLM-WILLIAMS: "The binding of plasmin-streptokinase complex to fibrin monomer-Sepharose".

Procédé perfectionné de préparation d'activateur du plasminogène, activateur ainsi obtenu et médicament le contenant

La présente invention concerne un procédé perfectionné de préparation d'un activateur tissulaire du plasminogène tel que décrit dans le brevet française no 2252842 déposé le 3 décembre 1973 au nom de la Demanderesse et dans les brevets équivalents, brevets américain No 3 998 947 et brevet anglais no 1 492 959, qui sont mentionnés ici comme références.

Dans ce qui suit, les termes "activateur tissulaire du plasminogène" ou "activateur du plasminogène" désignent essentiellement un activateur décrit dans les brevets précédents.

Le procédé de préparation dudit activateur tissulaire du plasminogène décrit dans les brevets précédents présente comme inconvénient de nécessiter de nombreuses étapes de reprises dans des solutions salines de concentration différente avant d'obtenir un produit présentant une bonne pureté.

Il a été mis au point un nouveau procédé pour la préparation à grande échelle de cet activateur du plasminogène faisant appel à l'affinité naturelle des activateurs tissulaires du plasminogène pour la fibrine. En effet, la fibrine possède la propriété de fixer sélectivement cet activateur du plasminogène au milieu d'autres protéines inactives et d'en réaliser ainsi très rapidement la séparation.

Ce procédé présente l'avantage de pouvoir être réalisé à grande échelle et ainsi d'obtenir un activateur du plasminogène possédant une activité spécifique sensiblement supérieure au produit faisant l'objet de la demande principale.

L'utilisation directe de la fibrine, telle qu'elle est décrite par certains auteurs, présente de nombreux inconvénients, dont les principaux sont de ne pas permettre un travail à grande échelle et d'autre part de libérer en même temps que l'activateur du plasminogène, des fragments solubles de fibrine possédant des propriétés indésirables.

Le procédé faisant l'objet de la présente invention présente l'avantage d'isoler de la fibrine des fragments solubles porteurs du site de fixation de l'activateur du plasminogène et de les fixer par covalence sur une matrice insoluble. Le support ainsi préparé est totalement insoluble. Il possède des propriétés mécaniques propres à une application industrielle et peut être réutilisé de nombreuses fois sans perte de ses propriétés.

Ledit activateur peut d'ailleurs être associé à un polysaccharide sulfaté comme cela est décrit dans le certificat d'addition no 2 310 133 déposé le 5 Mai 1975 et bénéficier de la potentialisation qui résulte de cette association.

La présente invention concerne donc un perfectionnement au procédé de préparation d'un activateur du plasminogène selon les brevets précédents, caractérisé en ce qu'il comporte au moins les étapes:

— d'adsorption sélective dudit activateur sur un support d'affinité spécifique constitué de fragments solubles de fibrine fixés par covalence sur une matrice insoluble, et
— d'élution de l'activateur à partir du support portant l'activateur adsorbé.

Dans un mode de mise en oeuvre préféré du procédé selon la présente invention, le support d'affinité spécifique est préparé par couplage covalent de fragments solubles isolés de la fibrine sur une matrice rigide pouvant être notamment soit un polymère de dextran activé par le bromure de cyanogène, soit un verre poreux, tel que de la silice poreuse aminée.

Les fragments solubles de fibrine sont de préférence les fragments solubles dans un tampon phosphate à pH 7,4 et contenant de l'urée, en particulier à une concentration de 6M.

L'un des avantages importants du procédé selon la présente invention est que l'élution de l'activateur peut être effectuée par un simple changement de pH de la solution mise en contact avec le complexe activateur + support.

Ainsi, dans un mode de mise en oeuvre préféré du procédé selon la présente invention l'adsorption sélective est effectuée à partir d'une phase aqueuse contenant l'activateur du plasminogène à un pH compris entre 6 et 8 par filtration de cette solution sur le support d'affinité spécifique préalablement placé dans une colonne de chromatographie. L'activateur du plasminogène est retenu dans ces conditions tandis les protéines inactives sont entraînées.

De même, dans un mode de mise en oeuvre préféré l'élution de l'activateur du plasminogène à partir de la fibrine est effectuée par une solution aqueuse à un pH compris entre 3 et 5; de préférence à l'aide d'un tampon acétate de potassium — acide acétique à un pH voisin de 4,2. Cette élution est effectuée de préférence à froid à une température comprise entre 0 et 10°C, de préférence à 40°C. Bien que l'on puisse envisager d'autres modes de mise en oeuvre de ce procédé on préfère traiter une solution clarifiée d'activateur du plasminogène en y ajoutant de la fibrine, le pH de cette solution aqueuse étant tamponné à pH 7,4 à l'aide d'un tampon phosphate. Dans ces conditions, la fibrine portant l'activateur du plasminogène adsorbé pourra être séparée par précipitation et de préférence par centrifugation.

Bien entendu, le support portant l'activateur du plasminogène adsorbé pourra si cela est nécessaire être lavée plusieurs fois à l'aide d'une solution aqueuse, en particulier d'un tampon phosphate pH 7,4 par exemple à froid afin d'éliminer toutes les protéines inactives.

La phase aqueuse à traiter peut être obtenue par un procédé quelconque, en particulier par ceux décrits dans les brevets précédents, et en particulier par:

a) extraction d'un broyat d'organes animaux avec une solution aqueuse à un pH compris entre 3 et 5;

b) relargage de la solution obtenue par addition d'un sel;

c) reprise du précipité obtenu par une solution aqueuse à un pH compris entre 7 et 9.

Dans un mode de mise en oeuvre préféré du procédé selon l'invention, la phase aqueuse à traiter est obtenue à partir d'une bouillie d'organes broyés et congelés dans un tampon acétate de potassium — acide acétique à pH 4,2. Dans ces conditions, on obtient l'extrait brut contenant l'activateur du plasminogène dans la phase aqueuse, les différents résidus pouvant être éliminés par passage sur un séparateur et/ou par centrifugation.

Afin de parfaire ce début de traitement la solution aqueuse peut être traitée par du sulfate d'ammonium cristallisé par exemple à une concentration de l'ordre de 250 g/l afin de recueillir un précipité concentré en activateur du plasminogène lequel après reprise dans le tampon phosphate à pH 7,4 constituera la phase à traiter par les étapes décrites précédement.

Enfin, l'eluat obtenu après séparation de la fibrine pourra être concentré par tout moyen approprié en particulier par dialyse ou ultrafiltration contre un tampon phosphate ou tout autre milieu compatible avec l'utilisation thérapeutique ultérieure.

D'autres caractéristiques du procédé selon la présente invention pourront être déterminées à partir de l'exemple de mise en oeuvre que sera décrit ci-après.

Comme cela a été décrit dans les brevets précédents les organes animaux utilisés sont de préférence choisis parmi les poumons, coeurs et reins de porcs, veaux, boeufs, chevaux, agneaux, moutons, ou les ovaires ou utérus de porcins, bovins et ovins. On préfère tout particulièrement utiliser les ovaires et les utérus de porcins.

Pour ce qui concerne la fibrine utilisée, elle peut être préparés par exemple à partir du sang d'animaux tels que le porc, le boeuf, le cheval ou le mouton et être soumise à plusieurs lavages préalables par de l'eau et par le tampon phosphate puis séchée. Cette fibrine est de préférence conservée à basse température sous emballage hermétique.

Exemple.

On prélève à l'abattoit des ovaires de truie qui sont immédiatement congelés à — 40°C et qui sont broyés à cette température. Ce broyat d'organes congelés constitue la matière première servant à la préparation de l'activateur tissulaire selon la présente invention.

A 100 kg d'organes broyés et congelés on ajoute 300 l d'un tampon acétate de potassium — acide acétique à pH 4,2 que l'on extrait pendant 4 heures à + 4°C.

On élimine le résidu d'organes par passage dans un séparateur solide-liquide en continu, puis on clarifie la phase surnageante par centrifugation continue à 15 000 tours/minute à + 4°C.

L'extrait aqueux ainsi clarifié est recueilli puis on lui ajoute du sulfate d'ammonium cristallisé à raison de 250 g/l. Après dissolution complète du sulfate d'ammonium on abandonne le mélange pendant environ 12 heures à froid afin de laisser se développer un précipité. Le précipité brut est recueilli par centrifugation continue à 15 000 tours/minute à + 4°C. Le surnageant est éliminé.

Le précipité brut qui contient l'activité est repris par 200 l. de tampon phosphate à pH 7,4 à froid pendant 4 heures, puis le mélange obtenu est clarifié par centrifugation à 15 000 tours/minute.

*Préparation du support d'affinité spécifique*

A partir de fibrine soluble, 1 000 g de fibrine lavée et séchée sont extraits par 5 000 ml de tampon phosphate, pH 7,4 contenant de l'urée 6M pendant deux heures á froid. Le résidu est éliminé par centrifugation et la fraction aqueuse contenant les fragments solubles de fibrine est recueillie.

Les fragmenns de fibrine portant les sites de fixation de l'activateur du plasminogène sont alors précipités de leur solution par le sulfate d'ammonium à 60% de saturation pendant 3 heures à froid.

Le précipité est recueilli par centrifugation puis remis en solution dans 5 000 ml de tampon phosphate pH 7,4 et dialysé contre 100 l du même tampon pendant 15 heures à + 4°C.

Les fragments solubles de fibrine sont alors lyophilisés avant d'être couplés par covalence sur une matrice insoluble.

Deux types de matrices sont utilisés indifféremment:

— des polymères de dextran activés par le bromure de cyanogène tel qu'on en trouve dans le commerce pour cet usage,

— des verres poreux du commerce sur lesquels des ligands protéiques peuvent également être couplés par covalence au moyen du glutaraldéhyde.

1) *Cas des polymères de dextran*

Le lyophilisat de ligand fibrine est repris dans 1 000 ml de tampon de couplage au $NaHCO_3$ 0,1 M pH 8,3 contenant NaCl 0,5M puis on mélange cette solution pendant deux heures à température ambiante avec 150 g de gel de dextran activé au CNBr préalablement gonflé dans 30 l d'HCl 1 mM et essoré.

Le gel est recueilli, essoré puis lavé par 10 l de tampon de couplage.

Les sites libres restant sur le gel sont saturés par une solution de glycocolle 1M à pH 8,5.

Le gel lavé sur lequel les ligands fibrine sont fixés constitue le support d'affinité spécifique. Il est introduit tel quel dans une colonne de chromatographie et équilibré avec du tampon phosphate pH 7,4.

2) *Cas des verres poreux*

On agite pendant une nuit à température ordinaire 10 g de silice poreuse aminée dans 2 000 ml d'une solution de glutaraldéhyde à 5% dans du tampon phosphate pH 7,4.

Le verre poreux activé est ensuite décanté et lavé plusieurs fois par le tampon phosphate pH 7,4.

Le lyophilisat de ligand fibrine est repris dans 2 000 ml de tampon phosphate pH 7,4 et agité pendant 3 heures à 4°C avec les 10 g de verre poreux activé.

Le support ainsi réalisé est lavé par du tampon phosphate pH 7,4 et les sites libres restants sont saturés par une solution 1M de glycocolle à pH 8,5.

Le support d'affinité spécifique est alors lavé, introduit dans une colonne de chromatographie et équilibré avec du tampon phosphate pH 7,4.

*Adsorption sélective de l'activateur du plasminogène*

La solution clarifiée d'activateur du plasminogène en tampon phosphate pH 7,4 est chromatographiée sur le support d'affinité spécifique décrit précédemment. Toute l'activité est ainsi retenue sur la colonne et l'effluent est éliminé.

La colonne est ensuite lavée pour éliminer complètement les protéines inactives non fixées. Un premier lavage est effectuée avec du tampon phosphate pH 7,4 puis un second lavage avec de l'eau distillée jusqu'à ce qu'aucune trace de protéines ne soit décelable dans l'effluent.

*Elution de l'activateur du plasminogène*

L'activité fixée sur le support est éluée au moyen d'un tampon acétate de potassium-acide acétique à pH 4,2. L'éluat est fractionné et l'activité mesurée dans chaque fraction.

Les fractions contenant l'activateur du plasminogène sont regroupées et concentrées au 1/5ème de leur volume par ultrafiltration sur une membrane coupant à un poids moléculaire de 10 000 daltons, à basse température. On obtient ainsi de 2 à 4 l de concentrat qui est neutralisé à pH 7,4 par NaOH diluée puis on dialyse pendant 18 heures contre du tampon acétate à pH 7,4 à froid pour éliminer les ions acétate.

La solution dialysée est alors stérilisée par filtration sur membrane de 0,2 $\mu$.

Un échantillon de filtrat stérile est prélevé pour contrôler la teneur en protéines par la méthode de LOWRY:

*Les protéines* sont dosées selon la méthode de LOWRY (O.H.), ROSEBROUGH (N.J.), FARR (A.L.) and RANDALL (R.J.), J. Biol. Chem. (1951); et l'activité de la solution est ensuite répartie en flacons stériles, et lyophilisée stérilement:

*L'activité* est déterminée par la méthode de mesure du temps de lyse d'un caillot standard dont le principe est le suivant:

— Un caillot est formé avec du fibrinogène riche en plasminogène et en présence l'activateur tissulaire qui se trouve ainsi répartie dans la masse de la fibrine. L'action de l'activateur sur le plasminogène provoque la libération de plasmine qui à son tour détruit la fibrine et provoque la lyse du caillot. Le temps de lyse est proportionnel à la quantité d'activateur présent et cette méthode est étalonnée avec une préparation standard d'Urokinase. Une relation linéaire entre le temps de lyse et l'activité existe entre 10 minutes et 16 minutes dans les conditions opératoires utilisées.

La solution obtenue est ensuite conditionnée stérilement et soumise à divers contrôles de stérilité d'activité d'innocuité.

Après lyophylisation le produit est stocké à +4°C où il conserve son activité pendant plusieurs mois.

Dans ces conditions on obtient un produit titrant entre $20.10^6$ et $50.10^6$ unités pour 100 kg d'organes frais d'un produit titrant entre 15 000 et 20 000 unités par mg de protéines.

Les propriétés pharmacologiques de l'activateur tissulaire obtenu par ce procédé sont identiques à celles décrites dans les brevets mentionnés précédemment.

Toutefois le degré de purification est supérieur et permet d'obtenir une activité spécifique comprise entre 15 000 et 20 000 unités par mg de protéines comme cela a été dit précédemment.

De plus, la grande affinité pour la fibrine de l'activateur du plasminogène ainsi isolé lui confère un très grand intérêt sur le plan thérapeutique pour le traitement des thromboses.

C'est pourquoi la présente invention concerne également un activateur du plasminogène obtenu par le procédé de la présente invention et son application à titre de médicament.

**Revendications**

1. Procédé de séparation d'un activateur tissulaire du plasminogène caractérisé en ce qu'il comporte au moins les étapes:
— d'adsorption sélective dudit activateur sur un support d'affinité spécifique constitué de fragments solubles de fibrine fixés par covalence sur une matrice insoluble; et
— d'élution de l'activateur á partir de la fibrine portant l'activateur adsorbé.

2. Procédé selon la revendication 1, caractérisé par le fait que la matrice insoluble utilisée est un polymère de dextrane activé par le bromure de cyanogène ou un verre poreux.

3. Procédé selon l'une des revendications 1 et 2, caractérisé en ce que l'adsorption sélective de l'activateur est conduite en solution aqueuse à un pH compris entre 6 et 8.

4. Procédé selon la revendication 3, caractérisé en ce que l'adsorption sélective est conduite en phase aqueuse en présence d'un tampon phosphate pH 7,4.

5. Procédé selon l'une des revendications 1 à 4, caractérisé en ce que l'élution de l'activateur est effectuée par mise en contact du support portant l'activateur adsorbé avec une phase aqueuse à pH compris entre 3 et 5.

6. Procédé selon la revendication 5, caractérisé en ce que l'élution de l'activateur à partir du support est effectuée par mise en contact du support portant l'activateur adsorbé avec un tampon acétate de potassium — acide acétique à pH 4,2.

7. Procédé selon l'une des revendications 1 à 6, caractérisé en ce que la séparation est effectuée à froid.

8. Activateur tissulaire du plasminogène obtenu par la mise en oeuvre du procédé selon l'une des revendications 1 à 7.

9. A titre de médicament nouveau un activateur tissulaire du plasminogène selon la revendication 8.

**Patentansprüche**

1. Verfahren zur Trennung eines Gewebeaktivators des Plasminogens, dadurch gekennzeichnet, daß es mindestens die Etappen einer selektiven Adsorption des genannten Aktivators auf einen Träger spezifischer Affinität, bestehend aus löslichen Fragmenten von einem durch kovalente Bindung auf eine unlösliche Matrize fixiertes Fibrin, und einer Elution des Aktivators, ausgehend von dem den adsorbierten Aktivator tragenden Fibrin, aufweist.

2. Verfahren nach Anspruch 1, dadurch gekennzeichnet, daß die verwendete unlösliche Matrize ein durch Bromcyan aktiviertes Dextran oder ein poröses Glas ist.

3. Verfahren nach einem der Ansprüche 1 und 2, dadurch gekennzeichnet, daß die selektive Adsorption des Aktivators in einer wäßrigen Lössung mit einem pH zwischen 6 und 8 durchgeführt wird.

4. Verfahren nach Anspruch 3, dadurch gekennzeichnet, daß die selektive Adsorption in einer wäßrigen Phase in Gegenwart eines Phosphatpuffers pH 7,4 durchgeführt wird.

5. Verfahren nach einem der Ansprüche 1 bis 4, dadurch gekennzeichnet, daß die Elution des Aktivators durch das Zusammenbringen des den adsorbierten Aktivator tragenden Trägers mit einer wäßrigen Phase mit einem pH zwischen 3 und 5 durchgeführt wird.

6. Verfahren nach Anspruch 5, dadurch gekennzeichnet, daß die von Träger ausgehende Elution des Aktivators durch Zusammenbringen des den adsorbierten Aktivator tragenden Trägers mit einem Kaliumazetat — Essigsäure-Puffer mit pH 4,2 — durchgeführt wird.

7. Verfahren nach einem der Ansprüche 1 bis 6, dadurch gekennzeichnet, daß die Trennung in kaltem Zustand durchgeführt wird.

8. Gewebeaktivator des Plasminogens der durch die Ausführung des Verfahrens nach einem der Ansprüche 1 bis 7 erhalten wird.

9. Als neues Medikament ein Gewebeaktivator des Plasminogens nach Anspruch 8.

**Claims**

1. A process for separating a tissular plasminogen activator, characterised in that it comprises at least the following steps:
— selective adsorption of said activator on a support of specific affinity composed of soluble fibrin fragments which are covalently fixed on an insoluble matrix; and
— elution of the activator from the fibrin carrying the adsorbed activator.

2. A process according to claim 1, characterised in that the insoluble matrix which is used is a dextran polymer activated by cyanogen bromide or a porous glass.

3. A process according to one of claims 1 and 2, characterised in that the selective adsorption of the activator is carried out in an aqueous solution at a pH of between 6 and 8.

4. A process according to claim 3, characterised in that the selective adsorption is carried out in an aqueous phase in the presence of a phosphate buffer of ph 7.4.

5. A process according to one of claims 1 to 4, characterised in that the elution of the activator is carried out by bringing the support carrying the adsorbed activator into contact with an aqueous phase at a pH of between 3 and 5.

6. A process according to claim 5, characterised in that the elution of the activator from the support is carried out by bringing the support carrying the adsorbed activator into contact with a potassium acetate-acetic acid buffer at pH 4.2.

7. A process according to one of claims 1 to 6, characterised in that separation is carried out in the cold.

8. A tissular plasminogen activator obtained by carrying out the process according to one of claims 1 to 7.

9. A tissular plasminogen activator according to claim 8 as a new medicine.